(19) Europäisches Patentamt
European Patent Office
Office européen des brevets

(11) **EP 2 991 544 B1**

(12) **FASCICULE DE BREVET EUROPEEN**

(45) Date de publication et mention
de la délivrance du brevet:
**21.06.2017  Bulletin 2017/25**

(51) Int Cl.:
***A61B 5/00*** *(2006.01)*   ***G01N 21/17*** *(2006.01)*

(21) Numéro de dépôt: **14721435.7**

(22) Date de dépôt: **24.03.2014**

(86) Numéro de dépôt international:
**PCT/FR2014/050679**

(87) Numéro de publication internationale:
**WO 2014/177779 (06.11.2014 Gazette 2014/45)**

(54) **PROCÉDÉ ET DISPOSITIF DE LOCALISATION D'AU MOINS UNE CIBLE DANS UN MILIEU ÉLECTROMAGNÉTIQUEMENT ABSORBANT**

VERFAHREN UND VORRICHTUNG ZUR ORTUNG VON MINDESTENS EINEM ZIEL IN EINER ELEKTROMAGNETISCH ABSORBIERENDEN UMGEBUNG

METHOD AND DEVICE FOR LOCATING AT LEAST ONE TARGET IN AN ELECTROMAGNETICALLY ABSORBENT ENVIRONMENT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité:  **02.05.2013   FR 1354072**

(43) Date de publication de la demande:
**09.03.2016   Bulletin 2016/10**

(73) Titulaires:
• **Centre National de la Recherche Scientifique
75794 Paris Cedex 16 (FR)**
• **Ecole Centrale de Marseille
13013 Marseille (FR)**
• **Université d'Aix Marseille
13284 Marseille Cedex 07 (FR)**

(72) Inventeurs:
• **DA SILVA, Anabela
F-13004 Marseille (FR)**
• **MENSAH, Serge
F-13012 Marseille (FR)**

(74) Mandataire: **Bonnet, Michel
Cabinet Bonnet
93, rue Réaumur - Boîte 10
75002 Paris (FR)**

(56) Documents cités:
**EP-A1- 1 935 346     US-A1- 2012 302 866**

**Description**

La présente invention concerne un procédé de localisation d'au moins une cible dans un milieu électromagnétiquement absorbant. Elle concerne également un programme d'ordinateur et un dispositif correspondants.

L'invention s'applique plus particulièrement à un procédé de localisation d'au moins une cible dans un milieu électromagnétiquement absorbant, comportant les étapes suivantes :

- émission d'au moins un signal excitateur électromagnétique à partir d'au moins une source,
- réception, par au moins un capteur acoustique, d'un signal acoustique résultant de l'émission de ce signal excitateur,
- détection, dans le signal acoustique reçu par le capteur acoustique, d'un instant de réception d'une réponse au signal excitateur, cette réponse résultant d'une perturbation acoustique due à une hétérogénéité électromagnétique de la cible dans le milieu, et
- estimation d'une distance entre la cible et le capteur acoustique à l'aide de cet instant de réception.

Le contexte de l'invention est donc celui de l'imagerie optoacoustique, dite également photoacoustique, ou thermoacoustique, appliquée à la localisation d'hétérogénéités (ou cibles) enfouies dans un milieu caractérisé par des propriétés électromagnétiques et acoustiques différentes de ces hétérogénéités. C'est par exemple le cas d'hétérogénéités telles que des tumeurs dans des tissus biologiques. L'imagerie optoacoustique ou thermoacoustique est ainsi en plein essor actuellement dans le domaine biomédical, en particulier parce qu'elle est réputée non invasive et non ionisante. Son principe est par exemple décrit dans l'article de C. Li et L. Wang, intitulé « Photoacoustic tomography and sensing in biomedicine », publié dans Physics in Medicine and Biology, vol. 54, pages R59-R97, 2009.

Les techniques actuelles d'imagerie optoacoustique ou thermoacoustique exploitent la génération instantanée d'une perturbation acoustique locale due à une hétérogénéité électromagnétique d'une cible dans un milieu électromagnétiquement absorbant et diffusant (si on se place en imagerie optoacoustique) ou diffractant (si on se place en imagerie thermoacoustique), lorsqu'un signal excitateur électromagnétique est émis depuis une source. Plus précisément, l'énergie électromagnétique émise, en étant partiellement absorbée dans le milieu observé, se dissipe thermiquement, ce qui crée des dilatations dans le milieu. En particulier, l'hétérogénéité électromagnétique de la cible crée localement une hétérogénéité de dilatation qui engendre à son tour la perturbation acoustique locale révélatrice de cette hétérogénéité.

Cette perturbation acoustique locale est détectée au niveau d'un capteur acoustique sous la forme d'une réponse avec un retard, par rapport à l'émission du signal excitateur, significatif de la distance entre la cible et le capteur acoustique. Cette distance peut donc être estimée à l'aide de l'instant de réception de la réponse précitée par le capteur acoustique. En effet, l'imagerie optoacoustique ou thermoacoustique utilise la propriété selon laquelle la génération de la perturbation acoustique au niveau de la cible se fait depuis la source à une vitesse voisine de celle de la lumière (vitesse de propagation électromagnétique), ou en tout cas très grande devant celle du son dans le milieu, alors que la propagation de la perturbation acoustique depuis la cible vers le capteur acoustique se fait à la vitesse du son. Par conséquent, en synchronisant la réception avec l'émission et en connaissant la vitesse du son dans le milieu, on déduit simplement la distance entre la cible et le capteur acoustique de la durée s'étant écoulée entre l'émission du signal excitateur et la réception de la réponse à ce signal.

Il est ainsi possible de localiser la cible dans l'espace tridimensionnel sur une sphère dont le centre est le capteur acoustique et dont le rayon est la distance estimée. Mais plusieurs émissions-réceptions sont nécessaires pour localiser plus précisément la cible. Ainsi les technologies actuelles impliquent une certaine complexité de capture et de traitement de signaux pour permettre une localisation de la cible. Comme en outre ces technologies sont généralement intégrées dans un principe plus global d'imagerie tomographique, leur complexité engendre également une plus grande complexité de reconstruction tomographique.

Une solution est de combiner la technologie optoacoustique ou thermoacoustique avec par exemple une technologie purement ultrasonore, pour limiter le nombre d'émissions/réceptions nécessaires. Une telle solution est proposée dans l'article de J. Zalev et al, intitulé « Clinical feasibility study of combined optoacoustic and ultrasonic imaging modality providing coregistered functional and anatomical maps of breast tumors », publié dans Photons Plus Ultrasound: Imaging and Sensing 2012, Proceedings of SPIE, vol. 8223, 2012. Elle reste complexe techniquement puisqu'elle combine deux technologies proches mais distinctes.

Par ailleurs, les technologies actuelles d'imagerie optoacoustique ou thermoacoustique limitent l'exploitation du signal acoustique résultant de l'émission du signal excitateur à l'inhomogénéité électromagnétique de la cible. Pour se concentrer sur cette propriété, en particulier quand le milieu dans lequel se trouve la cible est lui-même acoustiquement inhomogène, il est même enseigné de s'affranchir de ce problème en limitant la bande passante du capteur acoustique ou en effectuant des mesures acoustiques dans une configuration géométrique perpendiculaire à l'illumination du milieu. C'est ce qui est divulgué dans l'article de S. Ermilov et al, intitulé « Laser optoacoustic imaging system for détection of breast cancer », publié dans Journal of Biomedical Optics, vol. 14(2), pages 024007-1 à 024007-14, mars/avril 2009. Pourtant d'autres réponses au signal excitateur sont présentes dans le signal reçu par le capteur acoustique et mériteraient sans doute d'être exploitées plutôt que d'avoir recours à des combinaisons de technologies différentes. Ainsi par exemple, une inhomogénéité électromagnétique existe entre la source d'émission et le milieu, mais elle n'est pas exploitée dans les documents précités de l'art antérieur.

En revanche, dans la demande de brevet internationale WO 2011/096198 A1, l'inhomogénéité électromagnétique de la source par rapport au milieu est exploitée, plus précisément son inhomogénéité optique. Elle conduit à détecter, outre la réponse résultant d'une perturbation acoustique due à une hétérogénéité électromagnétique de la cible dans le milieu, une autre réponse résultant d'une perturbation acoustique due à l'hétérogénéité optique de la source par rapport au milieu. Cette autre réponse se propage à la vitesse du son dans le milieu, depuis la source vers le capteur acoustique. En connaissant la distance qui sépare la source du capteur acoustique, on en déduit une estimation de la vitesse du son dans le milieu. Cette solution permet donc de se passer d'une connaissance *a priori* de la vitesse du son dans le milieu pour estimer la distance séparant la cible du capteur acoustique. Mais là encore, plusieurs émissions-réceptions sont nécessaires pour localiser plus précisément la cible ce qui rend cette technologie complexe.

Le document EP 1 935 346 A1 divulgue les caractéristiques du préambule de la revendication 1.

Il peut ainsi être souhaité de prévoir un procédé de localisation d'au moins une cible dans un milieu qui permette de s'affranchir d'au moins une partie des problèmes et contraintes précités.

Il est donc proposé un procédé de localisation d'au moins une cible dans un milieu électromagnétiquement absorbant, comportant les étapes suivantes :

- émission d'au moins un signal excitateur électromagnétique à partir d'au moins une source,
- réception, par au moins un capteur acoustique, d'un signal acoustique résultant de l'émission de ce signal excitateur,
- détection, dans le signal acoustique reçu par le capteur acoustique, d'un premier instant de réception d'une première réponse au signal excitateur, cette première réponse résultant d'une perturbation acoustique due à une hétérogénéité électromagnétique de la cible dans le milieu,
- estimation d'une première distance entre la cible et le capteur acoustique à l'aide de ce premier instant de réception,

comportant en outre les caractéristiques suivantes :

- détection, dans le même signal acoustique reçu par le capteur acoustique, d'un second instant de réception d'une seconde réponse au signal excitateur, cette seconde réponse résultant d'une perturbation acoustique due à une hétérogénéité acoustique de la cible dans le milieu,
- estimation d'une seconde distance entre la source et la cible à l'aide de ce second instant de réception, et
- obtention d'une localisation de la cible à partir des première et seconde distances estimées.

En exploitant l'hétérogénéité acoustique de la cible dans le milieu, un procédé de localisation selon l'invention permet d'améliorer très significativement les technologies existantes. En effet, cela conduit à détecter, outre la première réponse résultant d'une première perturbation acoustique due à l'hétérogénéité électromagnétique de la cible dans le milieu, une seconde réponse résultant d'une seconde perturbation acoustique due à l'hétérogénéité acoustique de la cible. La seconde perturbation acoustique est générée au niveau de la source et se propage, depuis la source jusqu'à la cible, à la vitesse du son dans le milieu, puis continue à se propager, depuis la cible jusqu'au capteur acoustique, également à la vitesse du son dans le milieu. Par conséquent, en synchronisant la réception avec l'émission et en connaissant la vitesse du son dans le milieu, on déduit simplement la longueur du trajet source-cible-capteur de la durée s'étant écoulée entre l'émission du signal excitateur et la réception de la seconde réponse précitée. Connaissant en outre la distance entre la cible et le capteur grâce à la première réponse, on en déduit la distance entre la source et la cible à l'aide de l'instant de réception de la seconde réponse. On peut aussi déduire la distance entre la source et la cible directement à partir de la durée écoulée entre les premier et second instants de réception. Ainsi, avec une seule émission, on dispose de deux informations, la distance source-cible et la distance cible-capteur, pour localiser plus précisément la cible dans le milieu. A nombre d'émissions-réceptions constant, on obtient donc une meilleure localisation. En variante, pour une précision de localisation souhaitée de la cible, il devient possible de réduire le nombre d'émissions-réceptions.

De façon optionnelle :

- l'estimation de la distance entre la cible et le capteur acoustique se fait par multiplication d'un premier temps de vol de la première réponse entre la cible et le capteur acoustique avec une valeur prédéterminée de vitesse d'onde acoustique dans le milieu,
- le premier temps de vol est estimé en tant que temps écoulé entre l'instant d'émission du signal excitateur et le premier instant de réception.

De façon optionnelle également :

- l'estimation de la distance entre la source et la cible se fait par multiplication d'un second temps de vol de la seconde réponse entre la source et la cible avec la valeur prédéterminée de vitesse d'onde acoustique dans le milieu,
- le second temps de vol est estimé en tant que différence entre, d'une part, le temps écoulé entre l'instant d'émission du signal excitateur et le second instant de réception, et, d'autre part, le premier temps de vol.

De façon optionnelle également, la localisation de la cible dans l'espace tridimensionnel est obtenue par intersection de deux sphères, la première sphère ayant pour centre le capteur acoustique et pour rayon la première distance, la seconde sphère ayant pour centre la source et pour rayon la seconde distance.

De façon optionnelle également, le signal excitateur émis est un signal optique issu d'une source lumineuse de fréquence et/ou d'intensité modulées et les deux réponses au signal excitateur sont des réponses impulsionnelles résultant de deux perturbations acoustiques dues à une double hétérogénéité optique et acoustique de la cible dans le milieu.

De façon optionnelle également, un procédé de localisation d'au moins une cible selon l'invention peut comporter les étapes suivantes :

- émission de signaux excitateurs électromagnétiques à partir de plusieurs sources réparties en périphérie du milieu dans lequel est située la cible,
- réception, par plusieurs capteurs acoustiques répartis en périphérie du milieu dans lequel est située la cible, de signaux acoustiques résultant des émissions de ces signaux excitateurs, et
- reconstruction tomographique d'une image de la cible dans le milieu dans lequel elle est située, à l'aide des localisations obtenues de la cible.

De façon optionnelle également, les sources et les capteurs acoustiques sont répartis régulièrement sur un cercle autour du milieu dans lequel est située la cible.

De façon optionnelle également, le milieu électromagnétiquement absorbant est constitué d'un tissu biologique et la cible est une hétérogénéité dans ce tissu biologique.

Il est également proposé un programme d'ordinateur téléchargeable depuis un réseau de communication et/ou enregistré sur un support lisible par ordinateur et/ou exécutable par un processeur, comprenant des instructions pour l'exécution des étapes d'un procédé de localisation d'au moins une cible selon l'invention, lorsque ledit programme est exécuté sur un dispositif selon l'invention.

Il est également proposé un dispositif de localisation d'au moins une cible dans un milieu électromagnétiquement absorbant, comportant :

- au moins une source d'émission d'au moins un signal excitateur électromagnétique,
- au moins un capteur acoustique d'un signal acoustique résultant de l'émission de ce signal excitateur,
- un calculateur conçu pour détecter, dans le signal acoustique reçu par le capteur acoustique, un premier instant de réception d'une première réponse au signal excitateur, cette première réponse résultant d'une perturbation acoustique due à une hétérogénéité électromagnétique de la cible dans le milieu, et pour estimer une première distance entre la cible et le capteur acoustique à l'aide de ce premier instant de réception,

dans lequel le calculateur est en outre conçu pour :

- détecter, dans le même signal acoustique reçu par le capteur acoustique, un second instant de réception d'une seconde réponse au signal excitateur, cette seconde réponse résultant d'une perturbation acoustique due à une hétérogénéité acoustique de la cible dans le milieu,
- estimer une seconde distance entre la source et la cible à l'aide de ce second instant de réception, et
- fournir une localisation de la cible à partir des première et seconde distances estimées.

L'invention sera mieux comprise à l'aide de la description qui va suivre, donnée uniquement à titre d'exemple et faite en se référant aux dessins annexés dans lesquels :

- la figure 1 représente schématiquement la structure générale d'un dispositif de localisation de cible dans un milieu électromagnétiquement absorbant, selon un mode de réalisation de l'invention,
- la figure 2 illustre les étapes successives d'un procédé mis en oeuvre par le dispositif de la figure 1,
- la figure 3 illustre un exemple d'application du procédé de la figure 2,
- la figure 4 représente schématiquement la structure générale d'un exemple de système d'émission/réception pour le dispositif de la figure 1, et
- la figure 5 illustre l'intégration du procédé de la figure 2 dans un schéma plus global de reconstruction tomographique par imagerie optoacoustique ou thermoacoustique.

Dans ce qui suit, on se place dans le domaine de l'optoacoustique (ou photoacoustique) de façon purement illustrative et non limitative.

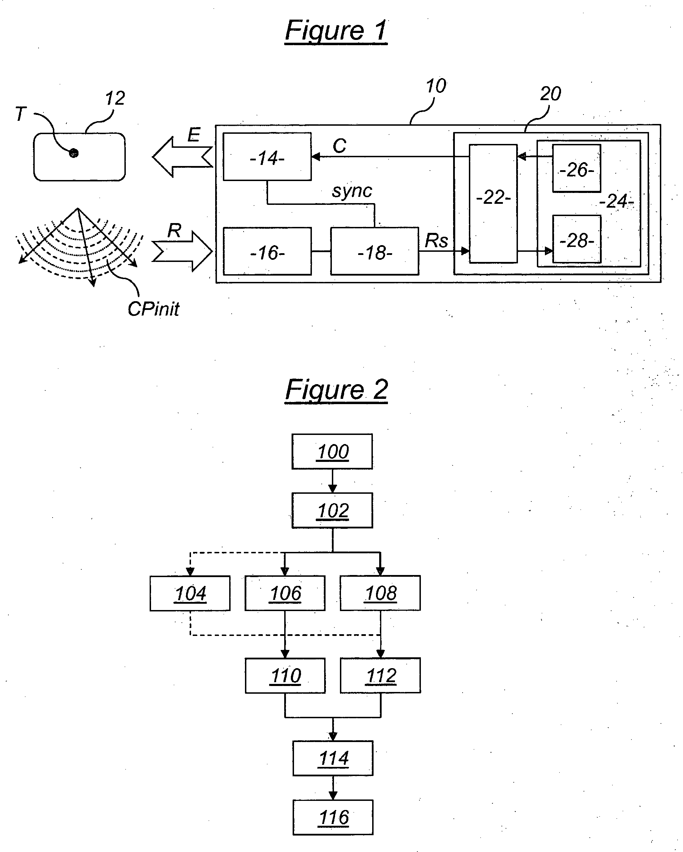

Le dispositif 10 représenté schématiquement sur la figure 1 est ainsi un dispositif d'imagerie optoacoustique permettant la localisation d'au moins une cible T située dans un milieu 12 optiquement absorbant et diffusant (puisque l'on se place en imagerie optoacoustique). La cible T, par exemple une tumeur dans un milieu observé 12 constitué d'un tissu biologique, présente une double hétérogénéité optique et acoustique dans le milieu 12 qui est par ailleurs homogène du point de vue de ces deux caractéristiques. Cette double hétérogénéité de la cible T est exploitée par le dispositif 10 pour permettre sa localisation, et éventuellement sa visualisation.

A cette fin, le dispositif de localisation 10 comporte un émetteur 14 comportant au moins une source d'émission d'au moins un signal excitateur électromagnétique E, plus précisément optique dans cet exemple. Cet émetteur 14 est conçu pour illuminer optiquement le milieu 12 et engendrer des perturbations acoustiques dues à la double hétérogénéité optique et acoustique de la cible T dans son milieu 12 et à l'hétérogénéité optique de la source d'émission par rapport au milieu 12. Ainsi, de façon connue en soi et selon les principes généraux de l'imagerie optoacoustique, l'émetteur 14 est une source lumineuse de fréquence et/ou d'intensité modulées, par exemple un émetteur laser pulsé de type Nd :Yag à 1064 nm, de largeur temporelle d'impulsion laser d'environ 3 ns, présentant un taux de répétition de 10 Hz et une puissance inférieure à 20 mJ/cm$^2$ si le milieu 12 est un tissu biologique. Compte tenu des propriétés optiques du milieu 12, il se crée instantanément, c'est-à-dire à une vitesse proche de celle de la lumière ou en tout cas très grande devant cette du son dans le milieu 12, une carte CPinit de distribution de pression initiale dans le milieu 12 révélatrice des dilatations mentionnées précédemment. Cette distribution de pression initiale engendre ensuite une onde de pression acoustique qui se propage à la vitesse du son selon les propriétés acoustiques du milieu 12. Si des éléments dans le milieu, tels que la cible T, sont optiquemerit et acoustiquement inhomogènes, des perturbations acoustiques correspondant à ces hétérogénéités sont détectables par réceptions de signaux acoustiques résultant de l'émission du signal excitateur E.

Le dispositif de localisation 10 comporte donc en outre un récepteur 16 comportant au moins un capteur acoustique d'un signal acoustique R résultant de l'émission du signal excitateur E. Le ou les capteurs acoustiques du récepteur 16 sont bien connus de l'homme du métier et ne seront pas détaillés. On peut notamment se reporter aux documents de l'état de la technique cités précédemment.

Le dispositif de localisation 10 comporte en outre une carte d'acquisition 18 permettant un prétraitement du signal reçu R, par exemple un filtrage et une numérisation, ainsi qu'une synchronisation du signal prétraité à l'aide d'un signal « sync » reçu de l'émetteur 14. Le signal reçu R comporte en effet un certain nombre de réponses impulsionnelles acoustiques à chaque impulsion optique émise par l'émetteur 14, chacune de ces réponses impulsionnelles étant, comme cela sera détaillé par la suite, due à une hétérogénéité électromagnétique, plus précisément optique dans cet exemple, dans le milieu 12. Il est donc important de synchroniser le signal reçu R par rapport au signal excitateur E pour permettre une estimation des instants de réception des réponses impulsionnelles acoustiques détectables dans le signal reçu R par rapport à l'instant d'émission du signal excitateur E.

Le dispositif de localisation 10 comporte en outre un calculateur 20 programmé pour commander l'émetteur 14 à l'aide d'un signal C en fonction d'une illumination souhaitée du milieu 12 et pour analyser le signal reçu par le récepteur 16 et prétraité par la carte d'acquisition 18. Ce signal reçu et prétraité est noté Rs en sortie de la carte d'acquisition 18.

Le calculateur 20 tel que représenté schématiquement sur la figure 1, comporte une unité de traitement 22 associée de façon classique à une mémoire 24 (par exemple une mémoire RAM).

L'unité de traitement 22 peut par exemple être mise en oeuvre dans un dispositif informatique tel qu'un ordinateur classique comportant un processeur associé à une ou plusieurs mémoires pour le stockage de fichiers de données et de programmes d'ordinateurs. L'unité de traitement 22 peut alors elle-même être considérée comme formée de ce processeur associé à la mémoire 24 agissant en tant que mémoire de stockage des instructions que le processeur exécute sous forme de programmes d'ordinateurs.

La mémoire 24 telle qu'illustrée sur la figure 1 comporte ainsi fonctionnellement deux programmes d'ordinateurs ou deux fonctions d'un même programme d'ordinateur 26 et 28. On notera en effet que les programmes d'ordinateurs 26 et 28 sont présentés comme distincts, mais cette distinction est purement fonctionnelle. Ils pourraient tout aussi bien être regroupés selon toutes les combinaisons possibles en un ou plusieurs logiciels. Leurs fonctions pourraient aussi être au moins en partie micro programmées ou micro câblées dans des circuits intégrés dédiés. Ainsi, en variante, le dispositif informatique mettant en oeuvre le calculateur 20 pourrait être remplacé par un dispositif électronique composé uniquement de circuits numériques (sans programme d'ordinateur) pour la réalisation des mêmes actions.

Le premier programme d'ordinateur 26 comporte des instructions pour l'exécution d'une génération du signal C par l'unité de traitement 22. Ce type de programme d'ordinateur est bien connu et ne sera pas détaillé.

Le second programme d'ordinateur 28 comporte des instructions pour l'exécution d'une analyse du signal Rs par l'unité de traitement 22. Plus précisément, ces instructions sont conçues pour détecter, par une analyse du signal Rs :

- un premier instant $t_1$ de réception d'une première réponse au signal excitateur E, cette première réponse résultant d'une première perturbation acoustique due à l'hétérogénéité optique de la cible T dans le milieu 12,
- un deuxième instant $t_2$ de réception d'une deuxième réponse au signal excitateur E, cette deuxième réponse résultant d'une deuxième perturbation acoustique due à l'hétérogénéité acoustique de la cible T dans le milieu 12, et éventuellement
- un troisième instant $t_3$ de réception d'une troisième réponse au signal excitateur E, cette troisième réponse résultant d'une troisième perturbation acoustique due à l'hétérogénéité optique de la source d'émission du signal excitateur E par rapport au milieu 12.

La détection optionnelle du troisième instant $t_3$ est par exemple réalisée conformément à l'enseignement du document WO 2011/096198 A1 précité. Elle permet de déterminer, sur connaissance *a priori* de la distance notée SD entre la source d'émission du signal excitateur E et le capteur du signal acoustique R, quelle est la vitesse moyenne v de propagation d'une onde acoustique dans le milieu 12. En notant $t_0$ l'instant d'émission du signal excitateur E, la vitesse v s'estime à l'aide de la relation suivante :

$$v = \frac{SD}{t_3 - t_0}.$$

En variante, la vitesse v peut être considérée comme connue *a priori,* de sorte que son estimation n'est pas nécessaire.

La détection du premier instant $t_1$ est réalisée conformément à l'enseignement du document WO 2011/096198 A1 ou des autres documents de l'état de la technique cités précédemment. La propriété, selon laquelle la génération de la première perturbation acoustique au niveau de la cible se fait depuis la source à une vitesse voisine de celle de la lumière alors que la propagation de la perturbation acoustique depuis la cible vers le capteur acoustique se fait à la vitesse du son, est alors exploitée pour effectuer le calcul suivant :

$$TD = v.(t_1 - t_0), \qquad \text{ou} \quad TD = \frac{SD}{t_3 - t_0}.(t_1 - t_0),$$

où TD est la distance entre la cible T et le capteur du signal acoustique R. Il est en effet considéré avec une très bonne approximation que la première perturbation acoustique est générée au niveau de la cible T à l'instant $t_0$ alors que la réponse acoustique qu'elle engendre est reçue par le capteur du signal acoustique R à l'instant $t_1$.

La détection du deuxième instant $t_2$ est réalisée en contradiction avec l'enseignement des documents de l'état de la technique cités précédemment. Aucun de ces documents n'exploite la propriété selon laquelle une perturbation (i.e. la deuxième perturbation précitée) est générée au niveau de la cible en raison de son hétérogénéité acoustique dans le milieu 12. Pire, certains documents de l'état de la technique enseignent même de filtrer le signal acoustique R de manière à supprimer, entre autre, cette deuxième perturbation, pour supprimer l'effet des inhomogénéités acoustiques dans le milieu 12 perçues d'une façon générale comme un artéfact à éliminer. Au contraire et conformément à l'invention, la propriété, selon laquelle la génération de la deuxième perturbation acoustique au niveau de la cible se fait depuis la source à la vitesse du son et se propage ensuite de la cible jusqu'au capteur également à la vitesse du son, est exploitée pour effectuer le calcul suivant :

$$ST + TD = v.(t_2 - t_0),$$

où ST est la distance entre la source d'émission du signal excitateur E et la cible T. La deuxième perturbation acoustique est en effet générée au niveau de la source à l'instant $t_0$ alors que la réponse acoustique qu'elle engendre est reçue par le capteur du signal acoustique R à l'instant $t_2$.

On déduit des deux calculs précédents une expression de la distance ST :

$$ST = v.(t_2 - t_1), \qquad \text{ou} \quad ST = \frac{SD}{t_3 - t_0}.(t_2 - t_1).$$

Par conséquent, après avoir détecté les instants $t_1$, $t_2$ et éventuellement $t_3$, les instructions du second programme d'ordinateur 28 sont conçues pour :

- estimer la distance TD entre la cible et le capteur récepteur du signal acoustique R à l'aide du premier instant de réception $t_1$ et éventuellement du troisième instant de réception $t_3$ si la vitesse v n'est pas connue, selon le calcul détaillé précédemment,
- estimer la distance ST entre la source d'émission du signal excitateur E et la cible à l'aide du premier instant de réception $t_1$, du deuxième instant de réception $t_2$, et éventuellement du troisième instant de réception $t_3$ si la vitesse v n'est pas connue, selon le calcul détaillé précédemment,
- fournir une localisation de la cible à partir des distances ST et TD précédemment estimées.

La fourniture de la localisation se fait d'une façon générale dans l'espace tridimensionnel par intersection de deux sphères, la première sphère ayant pour centre le capteur récepteur du signal acoustique R et pour rayon la distance TD, la seconde sphère ayant pour centre la source d'émission du signal excitateur E et pour rayon la distance ST. Elle peut se faire plus précisément et avantageusement dans un plan d'illumination du milieu 12, l'intersection des deux sphères devenant ainsi une intersection de deux cercles. Ces deux cercles ayant *a priori* deux points d'intersection, il subsiste un doute sur la localisation de la cible T. Ce doute peut être levé à l'aide d'une autre émission/réception ou plus simplement à l'aide de considérations géométriques, par exemple si l'un des deux points se situe à l'extérieur du milieu observé 12. Ainsi, dans un plan, la localisation de la cible peut être résolue sans ambiguïté à partir d'une seule émission de signal excitateur E. En revanche, dans l'espace tridimensionnel, une émission supplémentaire peut être nécessaire pour résoudre la localisation sans ambiguïté par intersection de trois sphères. On notera qu'en outre les milieux à hétérogénéités acoustiques induisent une incohérence des positions estimées : l'incertitude sur une position globale peut cependant être minimisée en multipliant les angles d'examen du milieu observé 12.

Un procédé de localisation de la cible T dans le milieu 12 mis en oeuvre par le dispositif 10 précédemment décrit va maintenant être détaillé en référence à la figure 2.

Au cours d'une première étape d'émission 100, le signal excitateur optique E est émis à l'instant $t_0$ à partir d'une source S de l'émetteur 14 sur réception par ce dernier du signal de commande C émis par l'unité de traitement 22 du calculateur 20.

Au cours d'une étape suivante de réception 102, le signal acoustique R résultant de cette émission est reçu, à partir de l'instant $t_0$ et au moins jusqu'aux instants $t_1$, $t_2$ et éventuellement $t_3$, par un capteur D du récepteur 16 puis prétraité par la carte d'acquisition 18 qui transmet le signal acoustique prétraité Rs à l'unité de traitement 22 du calculateur 20.

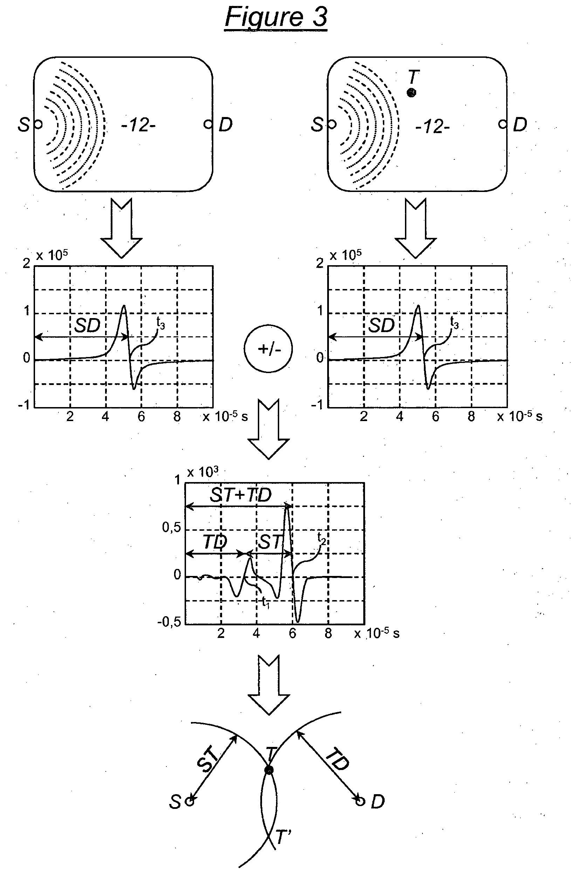

Au cours d'une étape optionnelle de détection 104 suivant l'étape 102, le troisième instant $t_3$ est détecté par exécution du second programme d'ordinateur 28. Le principe de cette détection sera détaillé en référence à la figure 3.

Au cours d'une étape de détection 106 suivant l'étape 102, le premier instant $t_1$ est détecté par exécution du second programme d'ordinateur 28. Le principe de cette détection est le même que celui de l'étape optionnelle 104.

Au cours d'une étape de détection 108 suivant l'étape 102, le deuxième instant $t_2$ est détecté par exécution du second programme d'ordinateur 28. Le principe de-cette détection est le même que celui de l'étape optionnelle 104.

Au cours d'une étape d'estimation 110 suivant l'étape 106, et éventuellement l'étape optionnelle 104 le cas échéant, la distance TD entre la cible T et le capteur acoustique D est estimée par exécution du second programme d'ordinateur 28 conformément au calcul détaillé précédemment.

Au cours d'une étape d'estimation 112 suivant l'étape 108, et éventuellement l'étape optionnelle 104 le cas échéant, la longueur ST+TD est estimée par exécution du second programme d'ordinateur 28 conformément au calcul détaillé précédemment.

Au cours d'une étape d'estimation 114 suivant les étapes 110 et 112, la distance ST entre la source S et la cible T est estimée par exécution du second programme d'ordinateur 28 par soustraction de TD à la longueur ST+TD. En variante, au lieu de procéder au calcul en deux étapes 112 et 114, on notera que la distance ST peut être directement calculée à l'aide des instants $t_1$ et $t_2$ (et éventuellement $t_3$) conformément au calcul détaillé précédemment.

Enfin, au cours d'une dernière étape de localisation 116, une localisation géométrique de la cible T par intersection de sphères ou de cercles est obtenue par exécution du second programme d'ordinateur 28, à partir des distances estimées ST et TD. De façon optionnelle et en variante, d'autres algorithmes de reconstruction tomographique peuvent être utilisés tels que par exemple une méthode de sommation de rétroprojections filtrées ou toute méthode algébrique connue.

Dans l'exemple illustré sur la figure 3, le procédé de la figure 2 mis en oeuvre par le dispositif 10 de la figure 1 est appliqué avec une seule source S d'émission de signal d'excitation E et un seul capteur acoustique D par souci de simplicité.

En partie supérieure gauche de la figure 3, les étapes 100, 102 et 104 sont appliquées au milieu 12 sans la cible T. Le signal Rs obtenu présente une réponse de forme impulsionnelle dans laquelle l'instant $t_3$ est détecté de la façon suivante. Cet instant est choisi comme correspondant au passage à zéro de la réponse impulsionnelle, celle-ci présentant un pic supérieur suivi d'un pic inférieur. Dans l'exemple particulier et expérimental illustré sur la figure 3, l'instant $t_3$ est détecté à $5,39.10^{-5}$ s, l'origine des temps étant choisie à $t_0$. Pour une vitesse de son v = 1485 m/s, on en déduit SD ≈ 8 cm. En pratique, c'est plutôt le calcul inverse qui est effectué. Connaissant par exemple *a priori* la distance SD de 8 cm entre la source S et le capteur D, on en déduit la valeur à 1485 m/s de la vitesse du son dans le milieu 12 grâce à la détection de $t_3 = 5,39.10^{-5}$ s.

En partie supérieure droite de la figure 3, les étapes 100, 102 et 104 sont appliquées au milieu 12 avec la cible T. Le signal Rs obtenu présente toujours une réponse de forme impulsionnelle dans laquelle l'instant $t_3$ peut être détecté de la même façon que précédemment. Mais, dans l'exemple illustré, les réponses impulsionnelles dues aux inhomogénéités optique et acoustique de la cible T ne sont pas visibles, celles-ci étant d'amplitudes très largement inférieures à celle due à l'inhomogénéité optique de la source.

Il est donc avantageux dans cet exemple de procéder à une soustraction entre les deux signaux Rs obtenus en partie supérieure gauche et droite de la figure 3. Concrètement, dans une application de détection et localisation d'une tumeur dans un tissu biologique, il suffit de disposer de signaux Rs de référence relatifs à un tissu sain qu'il convient de soustraire aux signaux captés pour un tissu biologique à tester.

Dans certaines applications et selon les contrastes de propriétés optiques et acoustiques entre les milieux, les trois réponses impulsionnelles peuvent être visibles sur le même signal acoustique de sorte qu'une soustraction avec un signal de référence n'est pas toujours nécessaire. Mais c'est en revanche généralement nécessaire pour des applications d'observation de tissus biologiques épais.

Dans l'exemple illustré, après la soustraction précitée, on obtient le signal Rs illustré en partie inférieure de la figure 3. Deux réponses impulsionnelles correspondant respectivement aux instants $t_1$ et $t_2$ sont maintenant bien visibles. La première, correspondant à l'instant $t_1$, présente un pic inférieur suivi d'un pic supérieur entre lesquels le passage à zéro détermine l'instant $t_1$. Dans l'exemple particulier et expérimental illustré sur la figure 3, l'instant $t_1$ est détecté à $3,39.10^{-5}$ s, l'origine des temps étant choisie à $t_0$. Pour une vitesse de son v = 1485 m/s, on en déduit TD ≈ 5 cm. La deuxième réponse impulsionnelle, correspondant à l'instant $t_2$, présente un pic supérieur suivi d'un pic inférieur entre lesquels le passage à zéro détermine l'instant $t_2$. Dans l'exemple particulier et expérimental illustré sur la figure 3, l'instant $t_2$ est détecté à $6,07.10^{-5}$ s, l'origine des temps étant choisie à $t_0$. Pour une vitesse de son v = 1485 m/s, on en déduit ST ≈ 4 cm.

Enfin, par reconstruction géométrique (intersection de deux sphères ramenée à une intersection de deux cercles si l'on se place dans un plan), la localisation de la cible T est identifiée à une inconnue près éventuellement entre deux localisations T et T'. Comme indiqué précédemment, cette inconnue peut être résolue par exemple si T' se trouve géométriquement à l'extérieur du milieu 12.

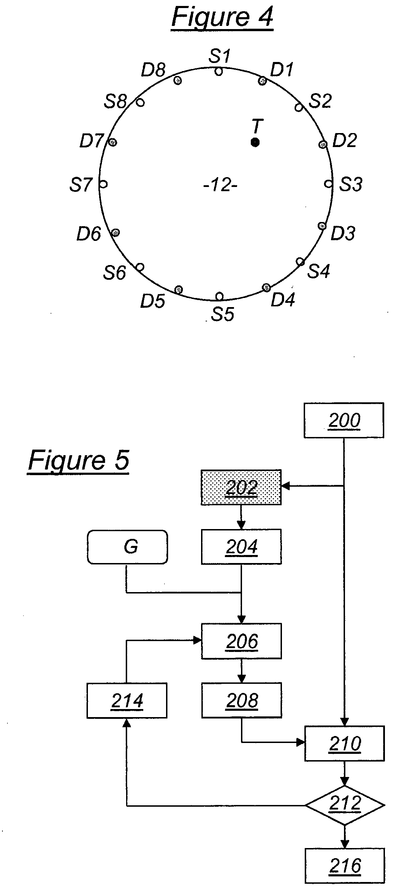

Comme illustré sur la figure 4, pour des applications d'imagerie par reconstruction tomographique, l'ensemble émetteur/récepteur 14, 16 peut comporter plusieurs sources et plusieurs capteurs acoustiques. Le milieu 12 observé est par exemple en forme de disque et plusieurs sources S1, S2, S3, S4, S5, S6, S7, S8 sont réparties régulièrement sur un cercle en périphérie de ce disque. De même, plusieurs capteurs acoustiques D1, D2, D3, D4, D5, D6, D7, D8 sont répartis régulièrement sur un cercle en périphérie de ce disque, intercalés entre les sources. Grâce à cette configuration de l'ensemble émetteur/récepteur 14, 16, il peut être procédé à une reconstruction tomographique optoacoustique d'une image de la cible T dans le milieu 12 dans lequel elle est située, à l'aide des localisations obtenues de la cible T par émissions/réceptions successives réalisées à l'aide de ces multiples sources et capteurs.

Un exemple de procédé d'imagerie par reconstruction tomographique intégrant un procédé de localisation selon l'invention est illustré sur la figure 5.

Au cours d'une étape 200, un ensemble de mesures par émissions/réceptions successives est réalisé à l'aide des sources optiques et des capteurs acoustiques de l'émetteur 14 et du récepteur 16.

Au cours d'une étape 202 suivant l'étape 200, les étapes 100 à 116 du procédé de la figure 2 sont exécutées pour chaque émission/réception de manière à fournir une localisation précise de la cible T dans le milieu 12.

Au cours d'une étape 204 suivant l'étape 202, la carte CPinit de distribution de pression initiale dans le milieu 12 est établie par simulation de l'illumination du milieu 12 par les sources optiques de l'émetteur 14, cette simulation intégrant, conformément à l'invention, la localisation de la cible T telle qu'estimée à l'étape 202.

A l'aide d'informations géométriques G sur les sources optiques de l'émetteur 14, sur les capteurs acoustiques du récepteur 16 et sur les contours extérieurs du milieu 12 illuminé, un modèle complet de ce milieu 12 incluant la cible T est établi lors d'une étape 206. De façon optionnelle et plus généralement, toute information *a priori* (morphologie, paramètres physiques ou physiologiques moyens, ...) peut être exploitée pour établir ce modèle.

Les mesures réalisées lors de l'étape 200 sont simulées sur le modèle établi à l'étape 206 au cours d'une étape suivante 208.

Les mesures simulées à l'étape 208 sont comparées aux mesures réelles de l'étape 200 au cours d'une étape 210. Cette comparaison se fait de façon connue en soi par minimisation d'une fonction d'erreur, par exemple une erreur quadratique.

Au cours d'une étape de test suivante 212, la fonction d'erreur est comparée à un seuil ε prédéterminé. Si elle reste supérieure à ce seuil, le procédé passe à une étape 214 de révision de la carte CPinit de distribution de pression initiale. Sinon, le procédé passe à une étape 216 d'enregistrement, et éventuellement d'affichage, d'une image représentant le milieu 12 avec la cible T correctement localisée, cette image étant définie sur la base du modèle établi à l'étape 206.

Au cours de l'étape 214, la révision de la carte CPinit de distribution de pression initiale se fait de façon connue en soi par optimisation de la fonction d'erreur. A l'issue de cette étape, le procédé reprend à l'étape 206 de manière à mettre à jour le modèle établi précédemment en fonction de la carte CPinit de distribution de pression initiale telle que révisée à l'étape 214.

Il apparaît clairement qu'un dispositif et un procédé de localisation tels que ceux décrits précédemment permettent de localiser plus efficacement une cible dans un milieu illuminé par une technologie optoacoustique. Notamment, les principes de détection proposés permettent de s'affranchir d'une combinaison de technologies différentes pour parvenir à une localisation, puis à une représentation, précise de cibles dans un milieu. Ils exploitent de façon optimale les signaux acoustiques reçus en réponse aux excitations optiques émises, en tirant parti des propriétés d'inhomogénéité optiques mais également acoustiques des cibles à détecter.

Les résultats sont particulièrement convaincants dans le domaine de l'imagerie médicale, pour la détection de tumeurs dans des tissus biologiques. En outre, en imagerie médicale, l'application d'un procédé de localisation selon l'invention est compatible avec l'utilisation d'agents de contrastes comme dans les autres technologies conventionnelles.

On notera par ailleurs que l'invention n'est pas limitée aux modes de réalisation décrits précédemment.

En particulier, la description a porté plus précisément sur la mise en oeuvre de l'invention dans le contexte d'une technologie d'acquisition optoacoustique. Mais il est bien évident que ses principes s'adaptent simplement et de façon connue en soi par l'homme du métier à une technologie d'acquisition thermoacoustique, notamment parce que l'hypothèse de la propagation bien plus rapide des ondes électromagnétiques dans le milieu comparée à celle du son est également vérifiée en technologie thermoacoustique.

En fait, la technologie thermoacoustique, comme l'optoacoustique, utilise des émetteurs d'ondes électromagnétiques et des récepteurs acoustiques. Ce qui distingue principalement ces deux technologies, ce sont les propriétés, de diffusion ou diffraction, exploitées du milieu observé en fonction des longueurs d'ondes émises. Lorsque les longueurs d'ondes émises engendrent un phénomène de diffusion dans le milieu, c'est l'équation de transfert radiatif (dite équation ETR) qui s'applique et qui est exploitée par la technologie optoacoustique. Lorsque les longueurs d'ondes émises engendrent un phénomène de diffraction dans le milieu, ce sont les équations de Maxwell qui s'appliquent et qui sont exploitées par la technologie thermoacoustique. Le fait d'engendrer un phénomène de diffusion ou de diffraction est une question de taille des constituants du milieu observé par rapport aux longueurs d'ondes émises. Ainsi par exemple, lorsque l'on souhaite observer un milieu constitué d'un tissu biologique, la technologie optoacoustique s'applique si l'émetteur émet des ondes dans le spectre de l'optique visible ou du proche infrarouge, car alors le milieu est diffusant, et la technologie thermoacoustique s'applique si l'émetteur émet des micro-ondes, car alors le milieu est diffractant. Lorsque l'on souhaite observer un milieu constitué d'un matériau manufacturé transparent, la technologie thermoacoustique s'applique si l'émetteur émet des ondes dans le spectre de l'optique visible, car alors le milieu est diffractant.

Plus précisément, la transposition de l'enseignement détaillé précédemment en technologie thermoacoustique se fait sur la base des considérations suivantes :

- le milieu à observer est diffractant et non pas diffusant,
- en ce qui concerne l'émission de signaux d'excitation, il convient de se placer dans la bonne gamme de longueurs d'ondes en fonction de la nature du milieu observé de manière à engendrer le phénomène de diffraction exploitable en technologie thermoacoustique, par exemple la gamme des micro-ondes pour l'examen de tissus biologiques et la gamme des ondes optiques visibles pour l'examen de matériaux transparents,
- les contrastes électromagnétiques sont à variation locale des propriétés diélectriques, par variation des parties réelle et imaginaire des constantes de permittivité et conductivité diélectrique du milieu observé, de sorte que seules les équations de propagation changent,
- les équations régissant la carte de distribution initiale de pression sont les équations de Maxwell et pas l'équation de transfert radiatif ETR.

Il apparaîtra plus généralement à l'homme de l'art que diverses modifications peuvent être apportées aux modes de réalisation décrits ci-dessus, à la lumière de l'enseignement qui vient de lui être divulgué. Dans les revendications qui suivent, les termes utilisés ne doivent pas être interprétés comme limitant les revendications aux modes de réalisation exposés dans la présente description, mais doivent être interprétés pour y inclure tous les équivalents que les revendications visent à couvrir du fait de leur formulation et dont la prévision est à la portée de l'homme de l'art en appliquant ses connaissances générales à la mise en oeuvre de l'enseignement qui vient de lui être divulgué.

**Revendications**

1. Procédé de localisation d'au moins une cible (T) dans un milieu (12) électromagnétiquement absorbant, comportant les étapes suivantes :

    - émission (100) d'au moins un signal excitateur électromagnétique (E) à partir d'au moins une source (S ; S1, S2, S3, S4, S5, S6, S7, S8),
    - réception (102), par au moins un capteur acoustique (D ; D1, D2, D3, D4, D5, D6, D7, D8), d'un signal acoustique (R) résultant de l'émission de ce signal excitateur (E),
    - détection (106), dans le signal acoustique reçu par le capteur acoustique, d'un premier instant ($t_1$) de réception d'une première réponse au signal excitateur, cette première réponse résultant d'une perturbation acoustique due à une hétérogénéité électromagnétique de la cible (T) dans le milieu (12),
    - estimation (110) d'une première distance (TD) entre la cible (T) et le capteur acoustique (D ; D1, D2, D3, D4, D5, D6, D7, D8) à l'aide de ce premier instant de réception ($t_1$),
    - détection (108), dans le même signal acoustique reçu par le capteur acoustique, d'un second instant ($t_2$) de réception d'une seconde réponse au signal excitateur, cette seconde réponse résultant d'une perturbation acoustique due à une hétérogénéité acoustique de la cible (T) dans le milieu (12),

    (**caractérisé en ce qu**'il comporte en outre les étapes suivantes:)

    - estimation (112, 114) d'une seconde distance (ST) entre la source (S ; S1, S2, S3, S4, S5, S6, S7, S8) et la cible (T) à l'aide de ce second instant de réception ($t_2$), et
    - obtention (116) d'une localisation de la cible (T) à partir des première (TD) et seconde (ST) distances estimées.

2. Procédé de localisation d'au moins une cible (T) selon la revendication 1, dans lequel :

    - l'estimation (110) de la distance (TD) entre la cible (T) et le capteur acoustique (D ; D1, D2, D3, D4, D5, D6, D7, D8) se fait par multiplication d'un premier temps de vol de la première réponse entre la cible (T) et le capteur acoustique (D ; D1, D2, D3, D4, D5, D6, D7, D8) avec une valeur prédéterminée de vitesse d'onde acoustique dans le milieu (12),
    - le premier temps de vol est estimé en tant que temps écoulé entre l'instant d'émission du signal excitateur (E) et le premier instant de réception ($t_1$).

3. Procédé de localisation d'au moins une cible (T) selon la revendication 2, dans lequel :

    - l'estimation (112, 114) de la distance (ST) entre la source (S ; S1, S2, S3, S4, S5, S6, S7, S8) et la cible (T) se fait par multiplication d'un second temps de vol de la seconde réponse entre la source (S ; S1, S2, S3, S4, S5, S6, S7, S8) et la cible (T) avec la valeur prédéterminée de vitesse d'onde acoustique dans le milieu (12),
    - le second temps de vol est estimé en tant que différence entre, d'une part, le temps écoulé entre l'instant d'émission du signal excitateur (E) et le second instant de réception ($t_2$), et, d'autre part, le premier temps de vol.

4. Procédé de localisation d'au moins une cible (T) selon l'une quelconque des revendications 1 à 3, dans lequel la localisation (116) de la cible (T) dans l'espace tridimensionnel est obtenue par intersection de deux sphères, la première sphère ayant pour centre le capteur acoustique (D ; D1, D2, D3, D4, D5, D6, D7, D8) et pour rayon la première distance (TD), la seconde sphère ayant pour centre la source (S ; S1, S2, S3, S4, S5, S6, S7, S8) et pour rayon la seconde distance (ST).

5. Procédé de localisation d'au moins une cible (T) selon l'une quelconque des revendications 1 à 4, dans lequel le signal excitateur émis (E) est un signal optique issu d'une source lumineuse de fréquence et/ou d'intensité modulées et les deux réponses au signal excitateur (E) sont des réponses impulsionnelles résultant de deux perturbations acoustiques dues à une double hétérogénéité optique et acoustique de la cible (T) dans le milieu (12).

6. Procédé de localisation d'au moins une cible (T) selon l'une quelconque des revendications 1 à 5, comportant les étapes suivantes :

    - émission (100) de signaux excitateurs électromagnétiques (E) à partir de plusieurs sources (S1, S2, S3, S4, S5, S6, S7, S8) réparties en périphérie du milieu (12) dans lequel est située la cible (T),
    - réception (102), par plusieurs capteurs acoustiques (D1, D2, D3, D4, D5, D6, D7, D8) répartis en périphérie du milieu (12) dans lequel est située la cible (T), de signaux acoustiques (R) résultant des émissions de ces signaux excitateurs (E), et

- reconstruction tomographique (216) d'une image de la cible (T) dans le milieu (12) dans lequel elle est située, à l'aide des localisations obtenues (116) de la cible (T).

7. Procédé de localisation d'au moins une cible (T) selon la revendication 6, dans lequel les sources (S1, S2, S3, S4, S5, S6, S7, S8) et les capteurs acoustiques (D1, D2, D3, D4, D5, D6, D7, D8) sont répartis régulièrement sur un cercle autour du milieu (12) dans lequel est située la cible (T).

8. Procédé de localisation d'au moins une cible (T) selon l'une quelconque des revendications 1 à 7, dans lequel le milieu (12) électromagnétiquement absorbant est constitué d'un tissu biologique et la cible (T) est une hétérogénéité electromagnétiques et acoustiques dans ce tissu biologique.

9. Programme d'ordinateur (26, 28) téléchargeable depuis un réseau de communication et/ou enregistré sur un support (24) lisible par ordinateur (20) et/ou exécutable par un processeur (22), **caractérisé en ce qu'**il comprend des instructions pour l'exécution des étapes d'un procédé de localisation d'au moins une cible (T) selon l'une quelconque des revendications 1 à 7, lorsque ledit programme est exécuté sur un dispositif selon la revendication 10.

10. Dispositif (10) de localisation d'au moins une cible (T) dans un milieu (12) électromagnétiquement absorbant, comportant :

- au moins une source (S ; S1, S2, S3, S4, S5, S6, S7, S8) d'émission d'au moins un signal excitateur électromagnétique (E),
- au moins un capteur acoustique (D ; D1, D2, D3, D4, D5, D6, D7, D8) d'un signal acoustique (R) résultant de l'émission de ce signal excitateur (E),
- un calculateur (20) conçu pour détecter, dans le signal acoustiques reçu par le capteur acoustique, un premier instant ($t_1$) de réception d'une première réponse au signal excitateur, cette première réponse résultant d'une perturbation acoustique due à une hétérogénéité électromagnétique de la cible (T) dans le milieu (12), et pour estimer une première distance (TD) entre la cible (T) et le capteur acoustique (D ; D1, D2, D3, D4, D5, D6, D7, D8) à l'aide de ce premier instant de réception ($t_1$).
- détecter, dans le même signal acoustique reçu par le capteur acoustique, un second instant ($t_2$) de réception d'une seconde réponse au signal excitateur, cette seconde réponse résultant d'une perturbation acoustique due à une hétérogénéité acoustique de la cible (T) dans le milieu (12),

**caractérisé en ce que** le calculateur (20) est en outre conçu pour:

- estimer une seconde distance (ST) entre la source (S ; S1, S2, S3, S4, S5, S6, S7, S8) et la cible (T) à l'aide de ce second instant de réception $t_2$), et
- fournir une localisation de la cible (T) à partir des première (TD) et seconde (ST) distances estimées.

**Patentansprüche**

1. Verfahren zur Ortung von mindestens einem Ziel (T) in einer elektromagnetisch absorbierenden Umgebung (12), umfassend die folgenden Schritte:

- Senden (100) von mindestens einem elektromagnetischen Erregersignal (E) aus mindestens einer Quelle (S; S1, S2, S3, S4, S5, S6, S7, S8),
- Empfangen (102), durch mindestens einen akustischen Sensor (D; D1, D2, D3, D4, D5, D6, D7, D8), eines akustischen Signals (R), das aus dem Senden dieses Erregersignals (E) resultiert,
- Detektieren (106), in dem von dem akustischen Sensor empfangenen akustischen Signal, eines ersten Empfangszeitpunkts ($t_1$) einer ersten Antwort auf das Erregersignal, wobei diese erste Antwort aus einer akustischen Störung aufgrund einer elektromagnetischen Heterogenität des Ziels (T) in der Umgebung (12) resultiert,
- Schätzen (110) einer ersten Distanz (TD) zwischen dem Ziel (T) und dem akustischen Sensor (D; D1, D2, D3, D4, D5, D6, D7, D8) mithilfe dieses ersten Empfangszeitpunkts ($t_1$),
- Detektieren (108), in dem gleichen von dem akustischen Sensor empfangenen akustischen Signal, eines zweiten Empfangszeitpunkts ($t_2$) einer zweiten Antwort auf das Erregersignal, wobei diese zweite Antwort aus einer akustischen Störung aufgrund einer akustischen Heterogenität des Ziels (T) in der Umgebung (12) resultiert,

**dadurch gekennzeichnet, dass** es des Weiteren die folgenden Schritte umfasst:

- Schätzen (112, 114) einer zweiten Distanz (ST) zwischen der Quelle (S; S1, S2, S3, S4, S5, S6, S7, S8) und dem Ziel (T) mithilfe dieses zweiten Empfangszeitpunkts ($t_2$), und
- Erhalten (116) einer Ortung des Ziels (T) aus der ersten (TD) und zweiten (ST) geschätzten Distanz.

2. Verfahren zur Ortung von mindestens einem Ziel (T) nach Anspruch 1, wobei:

- das Schätzen (110) der Distanz (TD) zwischen dem Ziel (T) und dem akustischen Sensor (D; D1, D2, D3, D4, D5, D6, D7, D8) durch Multiplizieren einer ersten Laufzeit der ersten Antwort zwischen dem Ziel (T) und dem akustischen Sensor (D; D1, D2, D3, D4, D5, D6, D7, D8) mit einem vorbestimmten Wert der Geschwindigkeit von akustischen Wellen in der Umgebung (12) erfolgt,
- die erste Laufzeit als Zeit, die zwischen dem Sendezeitpunkt des Erregersignals (E) und dem ersten Empfangszeitpunkt ($t_1$) verstrichen ist, geschätzt wird.

3. Verfahren zur Ortung von mindestens einem Ziel (T) nach Anspruch 2, wobei:

- das Schätzen (112, 114) der Distanz (ST) zwischen der Quelle (S; S1, S2, S3, S4, S5, S6, S7, S8) und dem Ziel (T) durch Multiplizieren einer zweiten Laufzeit der zweiten Antwort zwischen der Quelle (S; S1, S2, S3, S4, S5, S6, S7, S8) und dem Ziel (T) mit dem vorbestimmten Wert der Geschwindigkeit von akustischen Wellen in der Umgebung (12) erfolgt,
- die zweite Laufzeit als Differenz zwischen zum einen der Zeit, die zwischen dem Sendezeitpunkt des Erregersignals (E) und dem zweiten Empfangszeitpunkt ($t_2$) verstrichen ist, und zum anderen der ersten Laufzeit geschätzt wird.

4. Verfahren zur Ortung von mindestens einem Ziel (T) nach einem der Ansprüche 1 bis 3, wobei die Ortung (116) des Ziels (T) im dreidimensionalen Raum durch Sich-Schneiden von zwei Sphären erhalten wird, wobei die erste Sphäre als Mittelpunkt den akustischen Sensor (D; D1, D2, D3, D4, D5, D6, D7, D8) und als Radius die erste Distanz (TD) besitzt, wobei die zweite Sphäre als Mittelpunkt die Quelle (S; S1, S2, S3, S4, S5, S6, S7, S8) und als Radius die zweite Distanz (ST) besitzt.

5. Verfahren zur Ortung von mindestens einem Ziel (T) nach einem der Ansprüche 1 bis 4, wobei es sich bei dem gesendeten Erregersignal (E) um ein aus einer Lichtquelle stammendes optisches Signal mit modulierter Frequenz und/oder Intensität handelt, und es sich bei den zwei Antworten auf das Erregersignal (E) um Impulsantworten handelt, die aus zwei akustischen Störungen aufgrund einer zweifachen optischen und akustischen Heterogenität des Ziels (T) in der Umgebung (12) resultiert.

6. Verfahren zur Ortung von mindestens einem Ziel (T) nach einem der Ansprüche 1 bis 5, umfassend die folgenden Schritte:

- Senden (100) von elektromagnetischen Erregersignalen (E) aus mehreren Quellen (S1, S2, S3, S4, S5, S6, S7, S8), die in der Peripherie der Umgebung (12), in der sich das Ziel (T) befindet, verteilt sind,
- Empfangen (102), durch mehrere akustische Sensoren (D1, D2, D3, D4, D5, D6, D7, D8), die in der Peripherie der Umgebung (12), in der sich das Ziel (T) befindet, verteilt sind, von akustischen Signalen (R), die aus dem Senden dieser Erregersignale (E) resultieren, und
- tomographisches Rekonstruieren (216) eines Bildes des Ziels (T) in der Umgebung (12), in der es sich befindet, mithilfe der von dem Ziel (T) erhaltenen Ortungen (116).

7. Verfahren zur Ortung von mindestens einem Ziel (T) nach Anspruch 6, wobei die Quellen (S1, S2, S3, S4, S5, S6, S7, S8) und die akustischen Sensoren (D1, D2, D3, D4, D5, D6, D7, D8) regelmäßig auf einem Kreis um die Umgebung (12), in der sich das Ziel (T) befindet, verteilt sind.

8. Verfahren zur Ortung von mindestens einem Ziel (T) nach einem der Ansprüche 1 bis 7, wobei die elektromagnetisch absorbierende Umgebung (12) aus einem biologischen Gewebe besteht und es sich bei dem Ziel (T) um eine elektromagnetische und akustische Heterogenität in diesem biologischen Gewebe handelt.

**9.** Computerprogramm (26, 28), das aus einem Kommunikationsnetz herunterladbar und/oder auf einem von einem Computer (20) lesbaren Träger (24) gespeichert und/oder durch einen Prozessor (22) ausführbar ist, **dadurch gekennzeichnet, dass** es Anweisungen umfasst zum Ausführen der Schritte eines Verfahrens zur Ortung von mindestens einem Ziel (T) nach einem der Ansprüche 1 bis 7, wenn das Programm auf einer Vorrichtung nach Anspruch 10 ausgeführt wird.

**10.** Vorrichtung (10) zur Ortung von mindestens einem Ziel (T) in einer elektromagnetisch absorbierenden Umgebung (12), umfassend:

- mindestens eine Sendequelle (S; S1, S2, S3, S4, S5, S6, S7, S8) mindestens eines elektromagnetischen Erregersignals (E),
- mindestens einen akustischen Sensor (D; D1, D2, D3, D4, D5, D6, D7, D8) eines akustischen Signals (R), das aus dem Senden dieses Erregersignals (E) resultiert,
- einen Rechner (20), der zum Detektieren eines ersten Empfangszeitpunkts ($t_1$) einer ersten Antwort auf das Erregersignal in dem von dem akustischen Sensor empfangenen akustischen Signal ausgelegt ist, wobei diese erste Antwort aus einer akustischen Störung aufgrund einer elektromagnetischen Heterogenität des Ziels (T) in der Umgebung (12) resultiert, und zum Schätzen einer ersten Distanz (TD) zwischen dem Ziel (T) und dem akustischen Sensor (D; D1, D2, D3, D4, D5, D6, D7, D8) mithilfe dieses ersten Empfangszeitpunkts ($t_1$),
- Detektieren, in dem gleichen von dem akustischen Sensor empfangenen akustischen Signal, eines zweiten Empfangszeitpunkts ($t_2$) einer zweiten Antwort auf das Erregersignal, wobei diese zweite Antwort aus einer akustischen Störung aufgrund einer akustischen Heterogenität des Ziels (T) in der Umgebung (12) resultiert,

**dadurch gekennzeichnet, dass** der Rechner (20) des Weiteren ausgelegt ist zum:

- Schätzen einer zweiten Distanz (ST) zwischen der Quelle (S; S1, S2, S3, S4, S5, S6, S7, S8) und dem Ziel (T) mithilfe dieses zweiten Empfangszeitpunkts ($t_2$), und
- Liefern einer Ortung des Ziels (T) aus der ersten (TD) und zweiten (ST) geschätzten Distanz.

## Claims

**1.** A method for locating at least one target (T) in an electromagnetically absorbent environment (12), comprising the following steps:

- emitting (100) at least one electromagnetic excitation signal (E) from at least one source (S; S1, S2, S3, S4, S5, S6, S7, S8),
- receiving (102), by at least one acoustic sensor (D; D1, D2, D3, D4, D5, D6, D7, D8), an acoustic signal (R) resulting from the emission of this excitation signal (E),
- detecting (106), in the received acoustic signal by the acoustic sensor, a first time ($t_1$) of receipt of a first response to the excitation signal, this first response resulting from an acoustic disturbance caused by an electromagnetic heterogeneity of the target (T) in the environment (12),
- estimating (110) a first distance (TD) between the target (T) and the acoustic sensor (D; D1, D2, D3, D4, D5, D6, D7, D8) using this first time of receipt ($t_1$),
- detecting (108), in the same received acoustic signal by the acoustic sensor, a second time ($t_2$) of receipt of a second response to the excitation signal, this second response resulting from an acoustic disturbance caused by an acoustic heterogeneity of the target (T) in the environment (12),

**characterized in that** it further comprises the following steps:

- estimating (112, 114) a second distance (ST) between the source (S; S1, S2, S3, S4, S5, S6, S7, S8) and the target (T) using this second time of receipt ($t_2$), and
- obtaining (116) a location of the target (T) from the first (TD) and second (ST) estimated distances.

**2.** The method for locating at least one target (T) according to claim 1, wherein:

- the estimating (110) of the distance (TD) between the target (T) and the acoustic sensor (D; D1, D2, D3, D4, D5, D6, D7, D8) is carried out by multiplying a first flight time of the first response between the target (T) and the acoustic sensor (D; D1, D2, D3, D4, D5, D6, D7, D8) by a predetermined value of the acoustic wave speed

EP 2 991 544 B1

in the environment (12),
- the first flight time is estimated as being the elapsed time between the time of emission of the excitation signal (E) and the first time of receipt ($t_1$).

3. The method for locating at least one target (T) according to claim 2, wherein:

- the estimating (112, 114) of the distance (ST) between the source (S; S1, S2, S3, S4, S5, S6, S7, S8) and the target (T) is carried out by multiplying a second flight time of the second response between the source (S; S1, S2, S3, S4, S5, S6, S7, S8) and the target (T) by the predetermined value of the acoustic wave speed in the environment (12),
- the second flight time is estimated as being the difference between, on the one hand, the elapsed time between the time of emission of the excitation signal (E) and the second time of receipt ($t_2$), and, on the other hand, the first flight time.

4. The method for locating at least one target (T) according to any of claims 1 to 3, wherein the location (116) of the target (T) in the three-dimensional space is obtained by intersection of two spheres, with the first sphere having as a center the acoustic sensor (D; D1, D2, D3, D4, D5, D6, D7, D8) and as a radius the first distance (TD), with the second sphere having the source as a center (S; S1, S2, S3, S4, S5, S6, S7, S8) and the second distance (ST) as a radius.

5. The method for locating at least one target (T) according to any of claims 1 to 4, wherein the excitation signal emitted (E) is an optical signal coming from a light source with a modulated frequency and/or intensity and the two responses to the excitation signal (E) are pulse response resulting from two acoustic disturbances caused by a double optical and acoustic heterogeneity of the target (T) in the environment (12).

6. The method for locating at least one target (T) according to any of claims 1 to 5, comprising the following steps:

- emitting (100) electromagnetic excitation signals (E) from several sources (S1, S2, S3, S4, S5, S6, S7, S8) distributed at the periphery of the environment (12) wherein the target (T) is located,
- receiving (102), by several acoustic sensors (D1, D2, D3, D4, D5, D6, D7, D8) distributed at the periphery of the environment (12) wherein the target (T) is located, acoustic signals (R) resulting from the emissions of these excitation signals (E), and
- tomographic reconstruction (216) of an image of the target (T) in the environment (12) wherein it is located, using obtained locations (116) of the target (T).

7. The method for locating at least one target (T) according to claim 6, wherein the sources (S1, S2, S3, S4, S5, S6, S7, S8) and the acoustic sensors (D1, D2, D3, D4, D5, D6, D7, D8) are distributed regularly on a circle around the environment (12) wherein the target (T) is located.

8. The method for locating at least one target (T) according to any of claims 1 to 7, wherein the electromagnetically absorbent environment (12) is made of a biological tissue and the target (T) is an electromagnetic and acoustic heterogeneity in this biological tissue.

9. A computer program (26, 28) that can be downloaded from a communication network and/or recorded on a support (24) that can be read by a computer (20) and/or that can be executed by a processor (22), **characterized in that** it comprises instructions for the execution of the steps of a method for locating at least one target (T) according to any of claims 1 to 7, when said program is executed on a device according to claim 10.

10. A device (10) for locating at least one target (T) in an electromagnetically absorbent environment (12), comprising:

- at least one source (S; S1, S2, S3, S4, S5, S6, S7, S8) for emitting at least one electromagnetic excitation signal (E),
- at least one acoustic sensor (D; D1, D2, D3, D4, D5, D6, D7, D8) of an acoustic signal (R) resulting from the emission of this excitation signal (E),
- a calculator (20) designed to detect, in the received acoustic signal by the acoustic sensor, a first time ($t_1$) of receipt of a first response to the excitation signal, this first response resulting from an acoustic disturbance caused by an electromagnetic heterogeneity of the target (T) in the environment (12), and to estimate a first distance (TD) between the target (T) and the acoustic sensor (D; D1, D2, D3, D4, D5, D6, D7, D8) using this

14

first time of receipt ($t_1$),

- detect, in the same received acoustic signal by the acoustic sensor, a second time ($t_2$) of receipt of a second response to the excitation signal, this second response resulting from an acoustic disturbance caused by an acoustic heterogeneity of the target (T) in the environment (12),

**characterized in that** the calculator (20) is furthermore designed to:

- estimate a second distance (ST) between the source (S; S1, S2, S3, S4, S5, S6, S7, S8) and the target (T) using this second time of receipt ($t_2$), and
- provide a locating of the target (T) from the first (TD) and second (ST) estimated distances.

## Figure 1

## Figure 2

# Figure 3

## Figure 4

## Figure 5

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- WO 2011096198 A1

- EP 1935346 A1

### Littérature non-brevet citée dans la description

- **C. LI ; L. WANG.** Photoacoustic tomography and sensing in biomedicine. *Physics in Medicine and Biology,* 2009, vol. 54, R59-R97
- **J. ZALEV et al.** Clinical feasibility study of combined optoacoustic and ultrasonic imaging modality providing coregistered functional and anatomical maps of breast tumors. *Photons Plus Ultrasound: Imaging and Sensing 2012, Proceedings of SPIE,* 2012, vol. 8223

- **S. ERMILOV et al.** Laser optoacoustic imaging system for détection of breast cancer. *Journal of Biomedical Optics,* vol. 14 (2